# EUROPÄISCHE PATENTSCHRIFT

(11) **EP 0 688 535 B1**
(45) Veröffentlichungstag und Bekanntmachung des Hinweises auf die Patenterteilung: **19.02.1997**
(21) Anmeldenummer: 95106956.6
(22) Anmeldetag: 09.05.1995
(51) Int. Cl.: A61B 17/28

(54) **Chirurgisches Rohrschaftinstrument**
Tubular shaft surgical instrument
Instrument chirurgical à tige tubulaire

(30) Priorität: 21.06.1994 DE 4421584
(43) Veröffentlichungstag der Anmeldung: 27.12.1995
(73) Patentinhaber: AESCULAP AG, 78532 Tuttlingen (DE)
(72) Erfinder: Morales, Pedro, D-78532 Tuttlingen (DE); Wäschle, Andreas, D-78598 Königsheim (DE); Weisshaupt, Dieter, D-78194 Immendingen (DE); Zepf, Rudolf, D-78604 Rietheim-Weilheim (DE); Psalmon, Francois, F-23000 Gueret (FR)
(74) Vertreter: Hoeger, Stellrecht & Partner

(56) Entgegenhaltungen:
- DE-U- 9 318 814

## Beschreibung

Die Erfindung betrifft ein chirurgisches Rohrschaftinstrument mit einem Schaft, mit einer darin verschiebbar gelagerten Schub- und Zugstange, mit einem Griffteil und einem Handgriff zur Verschiebung der Schub- und Zugstange relativ zum Schaft, und mit Verbindungsmitteln, die das Griffteil einerseits und der Schaft sowie die Schub- und Zugstange andererseits lösbar miteinander verbinden, wobei der Schaft in einer inneren Hülse des Griffteils mittels eines in eine Ausnehmung des Schafts eintauchenden, in der inneren Hülse radial verschiebbaren Verriegelungskörpers in axialer Richtung festgelegt ist, dessen radiale Auswärtsbewegung durch einen mit der inneren Hülse zusammenwirkenden Anschlag begrenzt ist, der in eine die radiale Auswärtsbewegung des Verriegelungskörpers freigebende Position verschiebbar ist, und wobei die Schub- und Zugstande mit einem verdickten Ende in eine Aufnahmeöffnung einer Spannzange eingreift

Derartige chirurgische Rohrschaftinstrumente werden verwendet, um an schwer zugänglichen Körperstellen chirurgische Werkzeuge einzusetzen, beispielsweise Zangen oder Scheren, bei denen zwei Werkzeugteile gegeneinander bewegt werden müssen. Diese Bewegung wird durch die Relativbewegung des Schafts gegenüber der Schub- und Zugstange übertragen.

Ein gattungsgemäßes Rohrschaftinstrument ist in der DE 93 18 814 U1 beschrieben. Dort können der Schaft und die Schub- und Zugstange durch getrenntes Lösen der jeweiligen Verbindungen einzeln vom Griffteil abgetrennt werden. Dies ermöglicht zwar eine Zerlegbarkeit des Instruments, beim gleichzeitigen Auswechseln der Baueinheit aus Schaft und Schub- und Zugstange ist jedoch eine komplizierte Betätigung von zwei Verbindungseinrichtungen notwendig. Dies ist nicht nur umständlich, es kann dabei auch wertvolle Zeit verloren gehen, die bei einer Operation dringend benötigt wird.

Es ist Aufgabe der Erfindung, eine besonders günstige Konstruktion zu schaffen, mit der mit einem Handgriff der Schaft und die Schub- und Zugstange ausgewechselt werden können, beispielsweise wenn die Verwendung desselben Griffteils mit einem anderen chirurgischen Werkzeug gewünscht wird.

Diese Aufgabe wird bei einem chirurgischen Rohrschaftinstrument der eingangs beschriebenen Art erfindungsgemäß dadurch gelöst, der Anschlag und die Spannzange durch ein gemeinsames Betätigungsglied in ihre jeweiligen Freigabepositionen verschiebbar sind.

Es werden also getrennte Verriegelungsmittel für den Schaft und für die Schub- und Zugstange vorgesehen, und zwar für den Schaft ein Gesperre mit einem radial einschiebbaren Verriegelungskörper und für die Schub- und Zugstange eine Spannzange, die beide mit ein und demselben Betätigungsglied zu betätigen sind, so daß bei einer Betätigung beide Verbindungen gelöst werden. Damit wird es möglich, sowohl den Schaft als auch die Schub- und Zugstange mit einem Griff vom Griffteil zu lösen beziehungsweise wieder einzusetzen.

Dabei kann vorgesehen sein, daß die Ausnehmung des Schafts in Umfangsrichtung und in Längsrichtung des Schafts begrenzt ist, so daß durch den Eingriff des Verriegelungskörpers in die Ausnehmung eine Fixierung des Schafts in axialer Richtung und in Umfangsrichtung erfolgt.

Insbesondere können längs des Umfangs des Schafts mehrere Ausnehmungen angeordnet sein, dadurch kann der Schaft in unterschiedlicher Winkelstellung im Griffteil festgelegt werden.

Gemäß einer bevorzugten Ausführungsform wird vorgesehen, daß der Anschlag durch eine die Hülse umgebende, auf dieser verschiebbare äußere Hülse gebildet wird, die in axialem Abstand einen Bereich mit geringem Innendurchmesser und einen Bereich mit größerem Innendurchmesser aufweist.

Dabei ist es vorteilhaft, wenn die äußere Hülse durch eine Feder in eine Verriegelungsposition verschoben wird, in der der Bereich mit geringerem Innendurchmesser einen den Verriegelungskörper aufnehmenden Radialkanal der inneren Hülse umgibt. Die Feder verschiebt also die äußere Hülse automatisch in die Verriegelungsposition, nur gegen Wirkung dieser Feder kann eine Freigabe des Schafts erreicht werden.

Dabei ist es günstig, wenn die äußere Hülse gegen die Wirkung der Feder in das Griffteil einschiebbar ist. Allein eine solche Einschubbewegung führt also dazu, daß der Schaft vom Griffteil gelöst wird.

Besonders günstig ist es, wenn die äußere Hülse das die Spannzange verschiebende Betätigungsglied ist. Beim Verschieben der äußeren Hülse wird also automatisch auch die Spannzange geöffnet, ohne daß dazu weitere Handgriffe notwendig sind.

Dazu kann vorgesehen sein, daß die äußere Hülse eine Verlängerung aufweist, die an der Spannzange anliegt und diese verschiebt.

Günstig ist es, wenn die äußere Hülse in der Verriegelungsstellung einen Abstand von der Spannzange einhält und an dieser erst nach einer Verschiebung entgegen der Kraft der die äußere Hülse in die Verriegelungsposition verschiebenden Feder zur Anlage gelangt. Dadurch wird erreicht, daß beim Verschieben der äußeren Hülse nacheinander die Verriegelung des Schafts erfolgt und erst dann eine Freigabe der Spannzange.

Bei einer bevorzugten Ausführungsform kann vorgesehen sein, daß die Spannzange einen Innenteil mit federnd nach außen biegbaren Spannbacken und eine diesen umgebenden und gegenüber diesem axial verschiebbaren, hülsenförmigen Außenteil aufweist, der durch eine Feder in eine die Spannbacken an ihrem freien Ende umgebende Verriegelungsposition verschoben wird.

Es ist dabei vorteilhaft, wenn die Verlängerung der äußeren Hülse durch zwei diametral gegenüberliegende Wandabschnitte gebildet wird, die zwischen sich einen Zwischenraum in Form einer Radialnut bilden, und wenn diese Wandabschnitte stirnseitig an dem hülsenförmigen Außenteil zur Anlage gelangen. Die Verlängerungen greifen damit seitlich an der inneren Hülse vorbei und erreichen den hülsenförmigen Außenteil der Spannzange, dadurch wird wenig Raum benötigt, um die Bewegung der äußeren Hülse in tiefer gelegene Bereiche des Griffteils zu übertragen.

Vorteilhaft ist es, wenn die ebenen Innenseiten der Wandabschnitte flächig an einem Quersteg des Griffteils anliegen. Es ergibt sich dadurch eine Verdrehsicherung der äußeren Hülse. Außerdem ist es vorteilhaft, wenn der Quersteg ein Außengewinde trägt, mit dem er in ein Gewinde in einem Gehäuse des Griffteils einschraubbar ist. Insbesondere kann dabei der Quersteg mit einer die innere und die äußere Hülse umgebenden Kappe verbunden sein.

Weiterhin kann vorgesehen sein, daß der Quersteg eine zentrale Bohrung aufweist, die die innere Hülse aufnimmt und axial unverschieblich lagert. Damit ergibt sich eine Konstruktion, die an einem selbstständig in das Gehäuse einschraubbaren Bauteil, welches den Quersteg und die Kappe umfaßt, die gesamte Verriegelungsvorrichtung für den Rohrschaft aufnimmt, wobei dann seitlich am Quersteg die Verlängerungen überstehen, die im Inneren des Gehäuses die Spannzange betätigen.

Insbesondere durch diese günstige Ausgestaltung wird es möglich, daß gemäß einer bevorzugten Ausführungsform der

Erfindung das Griffteil die Form eines Rohrstücks hat, welches konzentrisch zum Schaft und zu der Schub- und Zugstange angeordnet ist.

Die nachfolgende Beschreibung bevorzugter Ausführungsformen der Erfindung dient im Zusammenhang mit der Zeichnung der näheren Erläuterung. Es zeigen:

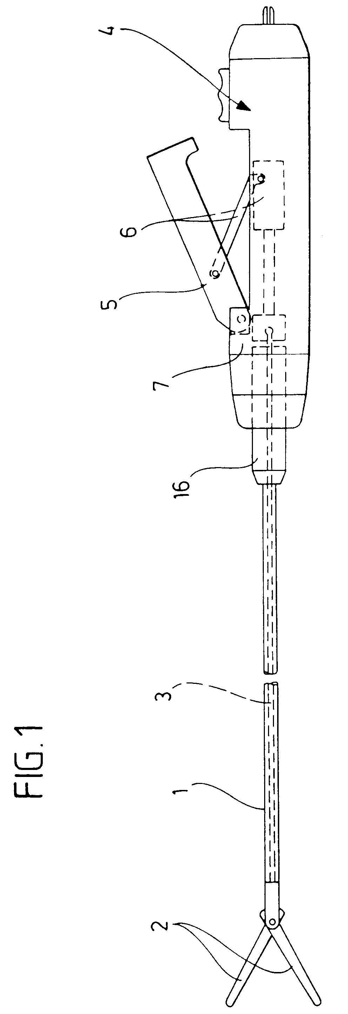

- Figur 1:: eine Seitenansicht eines Rohrschaftinstruments mit einem koaxial zum Rohrschaft angeordneten Griffteil;

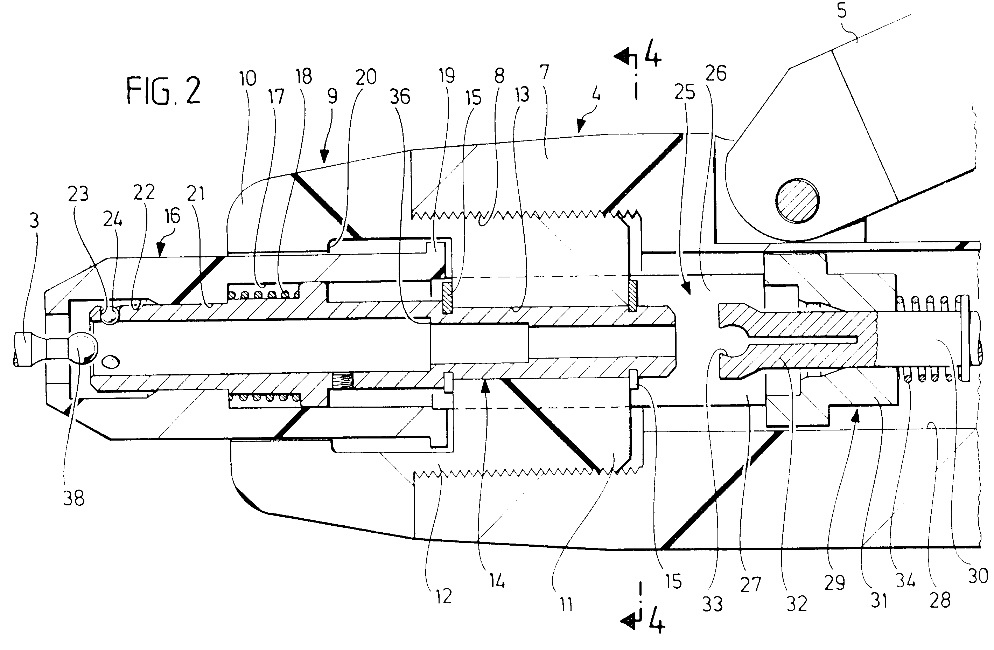

- Figur 2:: eine Längsschnittansicht des vorderen Griffteilbereichs des Rohrschaftinstruments der Figur 1 ohne eingesetzten Schaft und ohne eingesetzte Schub- und Zugstange;
- Figur 3:: eine Ansicht ähnlich Figur 2 mit eingesetztem Schaft und eingesetzter Schub- und Zugstange und

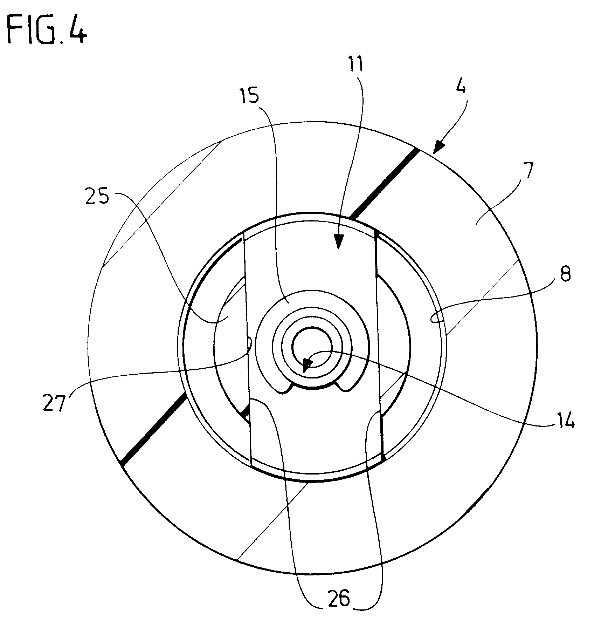

- Figur 4:: eine Schnittansicht längs Linie 4-4 in Figur 2.

Das in der Zeichnung dargestellte Rohrschaftinstrument umfaßt einen rohrförmigen Schaft 1, an dessen einem Ende zwei bewegbare Werkzeuge 2 gelagert sind, beispielsweise Zangenbacken oder Schneiden eines Schneidwerkzeugs. Im Inneren des Schafts 1 ist koaxial zu diesem eine Schub- und Zugstange 3 längsverschieblich gelagert, die über ein nicht eigens dargestelltes und an sich bekanntes Getriebe die Werkzeuge 2 gegensinnig verschwenkt, wenn die Schub- und Zugstange 3 gegenüber dem Schaft 1 verschoben wird.

Der Schaft 1 und die Schub- und Zugstange 3 sind an dem den Werkzeugen 2 gegenüberliegenden Ende in einem zylindrischen Griffteil 4 gehalten, welches konzentrisch zum Schaft 1 angeordnet ist und welches einen bewegbaren Handgriff 5 trägt, dessen Bewegung über geeignete Antriebsmittel 6 auf die Schub- und Zugstange 3 übertragen werden kann.

Das Griffteil 4 umfaßt ein zylindrisches Gehäuse 7, das an seiner Vorderseite offen ist und dort ein Innengewinde 8 trägt. In dieses Innengewinde 8 ist ein Kopfteil 9 eingeschraubt, welches eine Kappe 10 und einen Quersteg 11 umfaßt, der über zwei Schenkel 12 einstückig an die Kappe 10 angeformt ist. Der Quersteg 11 trägt an seinen beiden Stirnseiten ein Außengewinde 13, welches in das Innengewinde 8 eingeschraubt werden kann.

Außerdem nimmt der Quersteg 11 in einer zentralen Durchgangsbohrung 13 eine innere Hülse 14 auf, die durch beidseitig am Quersteg 11 anliegende Sprengringe 15 in axialer Richtung am Quersteg 11 festgelegt ist.

Auf der inneren Hülse 14 ist eine äußere Hülse 16 axial verschieblich gelagert, die zwischen sich und der inneren Hülse einen Ringraum 17 einschließt, in dem sich eine Schraubenfeder 18 befindet. Diese verschiebt die äußere Hülse 16 auf der inneren Hülse 14 nach außen, das heißt von dem Quersteg 11 weg; diese Verschiebebewegung wird durch eine nach außen abstehende Ringschulter 19 der äußeren Hülse 16 begrenzt, die an einer nach innen springenden Stufe 20 der Kappe 10 anschlägt.

Die äußere Hülse 16 ragt aus der Kappe 10 hervor und weist angrenzend an ihr freies Ende zwei Bereiche mit unterschiedlich großem Innendurchmesser auf. Ein Bereich 21 hat einen Innendurchmesser, der im wesentlichen dem Außendurchmesser der inneren Hülse 14 entspricht, ein in Richtung zum freien Ende der äußeren Hülse 16 daran anschließender Bereich 22 hat einen größeren Innendurchmesser. Bei entspannter Schraubenfeder 18 überdeckt der Bereich 21 mit geringerem Durchmesser das freie Ende der inneren Hülse 14, in diesem Teil befinden sich in der inneren Hülse 14 Radialkanäle 23, in die kugelförmige Verriegelungskörper 24 eingelegt sind. Auf der Innenseite verengen sich die Radialkanäle so, daß die Verriegelungskörper 24 nicht vollständig in den Innenraum der inneren Hülse 14 eintreten können, sie können jedoch teilweise in diesen Innenraum eintauchen. In dieser Position verbleiben die Verriegelungskörper 24, wenn die äußere Hülse 16 die Radialkanäle 23 mit ihrem Bereich 21 geringeren Innendurchmessers überdeckt, dagegen können die kugelförmigen Verriegelungskörper 24 im Radialkanal 23 radial nach außen verschoben werden, wenn die äußere Hülse 16 entgegen der Wirkung der Schraubenfeder 18 verschoben wird, so daß dann die Bereiche 22 mit größerem Innendurchmesser die Radialkanäle 23 überdecken.

Die äußere Hülse 16 trägt an ihrem in das Griffteil 4 weisenden Ende zwei Verlängerungen 25, die durch zwei diametral gegenüberliegende Wandabschnitte gebildet werden. Diese Wandabschnitte schließen zwischen sich einen radialnutenförmigen Zwischenraum 26 ein, in den der Quersteg 11 eintaucht. Die Verlängerungen 25 liegen dabei mit ihren ebenen Innenseiten 27 flächig an dem Quersteg 11 an, so daß dadurch die äußere Hülse gegen eine Verdrehung um ihre Längsachse gesichert wird.

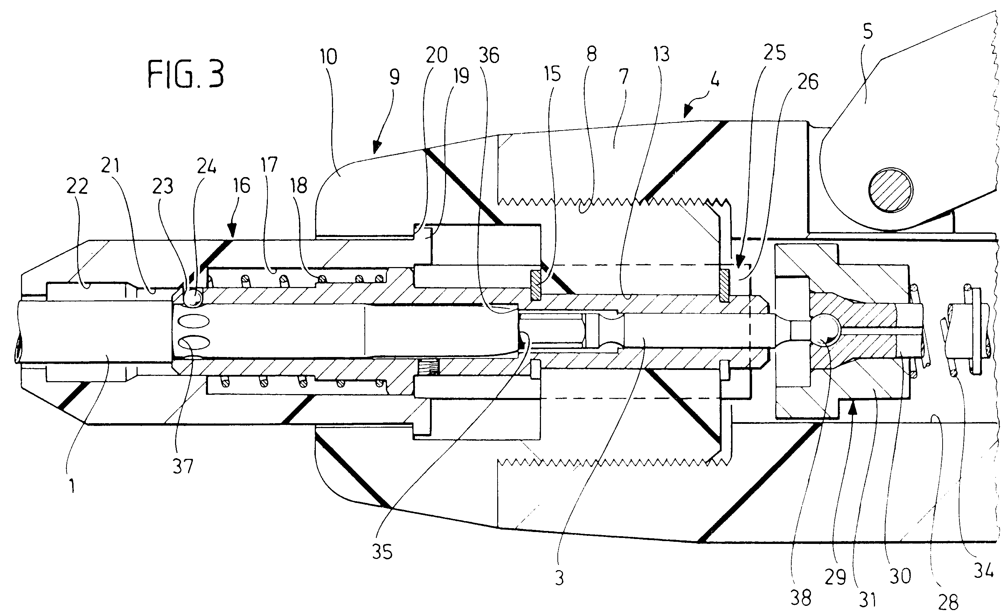

In einer sich an das Innengewinde 8 anschließenden Innenbohrung 28 des Griffteils 4 ist eine Spannzange 29 angeordnet mit einem Innenteil 30 und einem Außenteil 31. Das Innenteil 30 umfaßt mehrere elastisch nach außen biegbaren Spannbacken 32, die an der nach vorne gerichteten Stirnseite gemeinsam eine Aufnahmeöffnung 33 ausbilden. Auf dem Innenteil 30 ist das hülsenförmige Außenteil 31 in axialer Richtung verschiebbar und wird durch eine das Innenteil 30 umgebende Schraubenfeder 34 in eine Verriegelungsstellung verschoben, in der die Spannbacken 32 nach innen gedrückt und an einem elastischen Aufbiegen gehindert werden (Figur 3). Gegen die Wirkung der Schraubenfeder 34 läßt sich das hülsenförmige Außenteil 31 so weit verschieben, bis die Spannbacken 32 freigegeben werden und elastisch nach außen aufgebogen werden können, so daß der Eintritt in die Aufnahmeöffnung 33 elastisch erweitert werden kann.

Die Aufnahmeöffnung 33 befindet sich in geringem Abstand vom griffteilseitigen Ende der inneren Hülse 14, das hülsenförmige Außenteil 31 liegt dem Ende der Verlängerungen 25 gegenüber.

Beim Einschieben der äußeren Hülse 16 in das Griffteil 4 legen sich daher die Verlängerungen 25 an das hülsenförmige Außenteil 31 an und verschieben dieses entgegen der Wirkung der Schraubenfeder 34. Dadurch wird die Spannzange 29 freigegeben.

Der Außendurchmesser des Schafts 1 entspricht dem Innendurchmesser der inneren Hülse 14, so daß dieser in die innere Hülse 14 geführt eingeschoben werden kann, bis seine Stirnkante 35 an einem Absatz 36 der inneren Hülse 14 anschlägt. In dieser Position liegen mehrere in radialer Richtung verteilt angeordnete Ausnehmungen 37 im Schaft 1 den Radialkanälen 23 gegenüber, so daß die Verriegelungskörper 24 im Radialkanal 23 in die Ausnehmungen 37 eintreten können.

Nach hinten steht aus dem Schaft 1 die Schub- und Zugstange 3 hervor und endet in einem kugelförmigen Kopf 38. Dieser Kopf 38 steht aus der inneren Hülse 14 hervor und wird in der Aufnahmeöffnung 33 der Spannzange 29 aufgenommen.

Um Schaft 1 und Schub- und Zugstange 3 in das Griffteil 4 einzusetzen, ist es notwendig, die äußere Hülse 16 auf der inneren Hülse 14 entgegen der Wirkung der Schraubenfeder 18 in das Griffteil 4 einzuschieben, so daß einerseits die Verriegelungskörper 24 in den Radialkanälen 23 freigegeben werden und daß andererseits durch Anlage der Verlängerungen 25 an dem hülsenförmigen Außenteil 31 der Spannzange 29 die Spannzange geöffnet wird. Es ist dann möglich, Schaft und Schub- und Zugstange gemeinsam in das Instrument einzuführen, bis der Kopf 38 in die Aufnahmeöffnung 33 der Spannzange 29 eingreift und bis die Ausnehmungen 37 im Schaft 1 den Ringkanälen 23 gegenüberstehen. Läßt man nun die äußere Hülse los, so wird diese unter der Wirkung der Schraubenfeder 18 in ihre Verriegelungsstellung geschoben, dabei werden die Verriegelungskörper 24 dauerhaft in die Ausnehmungen 37 eintauchend gehalten. Gleichzeitig kann die Schraubenfeder 34 den hülsenförmigen Außenteil 31 der Spannzange 29 in die Verriegelungsstellung verschieben und somit den Kopf 38 in der Aufnahmeöffnung 33 dauerhaft festlegen.

Zum Lösen dieser Kupplung muß nur wieder die äußere Hülse 16 auf der inneren Hülse 14 in das Griffteil eingeschoben werden, dann sind die genannten Verriegelungen automatisch gelöst.

Obwohl die Spannzange 29 durch die äußere Hülse 16 der Schaftverriegelung betätigt wird, ist es ohne weiteres möglich, die Schaftverriegelung aus dem Griffteil zu entfernen und gegebenenfalls auszutauschen. Es genügt dazu, das Kopfteil 9 aus dem Gehäuse 7 herauszuschrauben, an diesem Kopfteil ist die gesamte Schaftverriegelung festgelegt. Es ist jetzt beispielsweise möglich, eine Schaftverriegelung für einen Schaft eines anderen Durchmessers einzusetzen, auch in diesem Falle kann dieselbe Spannzange 29 für die Schub- und Zugstange verwendet werden.

## Patentansprüche

1. Chirurgisches Rohrschaftinstrument mit einem Schaft (1), mit einer darin verschiebbar gelagerten Schub- und Zugstange (3), mit einem Griffteil (4) und einem Handgriff (5) zur Verschiebung der Schub- und Zugstange relativ zum Schaft, und mit Verbindungsmitteln, die das Griffteil einerseits und den Schaft sowie die Schub- und Zugstange andererseits lösbar miteinander verbinden, wobei der Schaft in einer inneren Hülse (14) des Griffteils mittels eines in eine Ausnehmung (37) des Schafts eintauchenden, in der inneren Hülse radial verschiebbaren Verriegelungskörpers (24) in axialer Richtung festgelegt ist, dessen radiale Auswärtsbewegung durch einen mit der inneren Hülse zusammenwirkenden Anschlag (21) begrenzt ist, der in eine die radiale Auswärtsbewegung des Verriegelungskörpers freigebende Position verschiebbar ist, und wobei die Schub- und Zugstange mit einem verdickten Ende in eine Aufnahmeöffnung (33) einer Spannzange eingreift, dadurch gekennzeichnet, daß der Anschlag (21) und die Spannzange (29) durch ein gemeinsames Betätigungsglied in ihre jeweiligen Freigabepositionen verschiebbar sind.

2. Chirurgisches Rohrschaftinstrument nach Anspruch 1, dadurch gekennzeichnet, daß der Verriegelungskörper (24) eine Kugel ist.

3. Chirurgisches Rohrschaftinstrument nach Anspruch 1 oder 2, dadurch gekennzeichnet, daß die Ausnehmung (37) des Schafts (1) in Umfangsrichtung und in Längsrichtung des Schafts (1) begrenzt ist, so daß durch den Eingriff des Verriegelungskörpers (24) in die Ausnehmung (37) eine Fixierung des Schafts (1) in axialer Richtung und in Umfangsrichtung erfolgt.

4. Chirurgisches Rohrschaftinstrument nach Anspruch 3, dadurch gekennzeichnet, daß längs des Umfangs des Schafts (1) mehrere Ausnehmungen (37) angeordnet sind.

5. Chirurgisches Rohrschaftinstrument nach einem der voranstehenden Ansprüche, dadurch gekennzeichnet, daß der Anschlag durch eine die innere Hülse (14) umgebende, auf dieser verschiebbare äußere Hülse (16) gebildet wird, die in axialem Abstand einen Bereich (21) mit geringem Innendurchmesser und einen Bereich (22) mit größerem Innendurchmesser aufweist.

6. Chirurgisches Rohrschaftinstrument nach Anspruch 5, dadurch gekennzeichnet, daß die äußere Hülse (16) durch eine Feder (18) in eine Verriegelungsposition verschoben wird, in der der Bereich (21) mit geringerem Innendurchmesser einen den Verriegelungskörper (24) aufnehmenden Radialkanal (23) der inneren Hülse (14) umgibt.

7. Chirurgisches Rohrschaftinstrument nach Anspruch 6, dadurch gekennzeichnet, daß die äußere Hülse (16) gegen die Wirkung der Feder (18) in das Griffteil (4) einschiebbar ist.

8. Chirurgisches Rohrschaftinstrument nach einem der Ansprüche 5 bis 7, dadurch gekennzeichnet, daß die äußere Hülse (16) das die Spannzange (29) verschiebende Betätigungsglied ist.

9. Chirurgisches Rohrschaftinstrument nach Anspruch 8, dadurch gekennzeichnet, daß die äußere Hülse (16) eine Verlängerung (25) aufweist, die an der Spannzange (29) anliegt und diese verschiebt.

10. Chirurgisches Rohrschaftinstrument nach einem der Ansprüche 8 oder 9, dadurch gekennzeichnet, daß die äußere Hülse (16) in der Verriegelungsstellung einen Abstand von der Spannzange (29) einhält und an dieser erst nach einer Verschiebung entgegen der Kraft der die äußere Hülse (16) in die Verriegelungsposition verschiebenden Feder (18) zur Anlage gelangt.

11. Chirurgisches Rohrschaftinstrument nach einem der voranstehenden Ansprüche, dadurch gekennzeichnet, daß die Spannzange (29) einen Innenteil (30) mit federnd nach außen biegbaren Spannbacken (32) und eine diesen umgebenden und gegen über diesem axial verschiebbaren, hülsenförmigen Außenteil (31) aufweist, der durch eine Feder (34) in eine die Spannbacken (32) an ihrem freien Ende umgebende Verriegelungsposition verschoben wird.

12. Chirurgisches Rohrschaftinstrument nach Anspruch 9 und 11, dadurch gekennzeichnet, daß die Verlängerung (25) der äußeren Hülse (16) durch zwei diametral gegenüberliegende Wandabschnitte gebildet wird, die zwischen sich einen Zwischenraum (26) in Form einer Radialnut bilden, und daß diese Wandabschnitte stirnseitig an dem hülsenförmigen Außenteil (31) zur Anlage gelangen.

13. Chirurgisches Rohrschaftinstrument nach Anspruch 12, dadurch gekennzeichnet, daß die ebenen Innenseiten (27) der Wandabschnitte flächig an einem Quersteg (11) des Griffteils (4) anliegen.

14. Chirurgisches Rohrschaftinstrument nach Anspruch 13, dadurch gekennzeichnet, daß der Quersteg (11) ein Außengewinde trägt, mit dem er in ein Innengewinde (8) in einem Gehäuse (7) des Griffteils (4) einschraubbar ist.

15. Chirurgisches Rohrschaftinstrument nach Anspruch 14, dadurch gekennzeichnet, daß der Quersteg (11) mit einer die innere und die äußere Hülse (14 beziehungsweise 16) umgebenden Kappe (10) verbunden ist.

16. Chirurgisches Rohrschaftinstrument nach einem der Ansprüche 13 bis 15, dadurch gekennzeichnet, daß der Quersteg (11) eine zentrale Bohrung (13) aufweist, die die innere Hülse (14) aufnimmt und axial unverschieblich lagert.

17. Chirurgisches Rohrschaftinstrument nach einem der voranstehenden Ansprüche, dadurch gekennzeichnet, daß das Griffteil (4) die Form eines Rohrstücks hat, welches konzentrisch zum Schaft (1) und zu der Schub- und Zugstange (3) angeordnet ist.

## Claims

1. A tubular-shaft surgical instrument comprising a shaft (1), a push-pull rod (3) displaceably mounted therein, a grip (4) and a handle (5) for displacing the push-pull rod relative to the shaft, and connecting means detachably connecting the grip on the one hand and the shaft and the push-pull rod on the other, wherein the shaft is secured in an inner sleeve (14) of the grip in the axial direction by means of a locking member (24) radially displaceable in the inner sleeve and projecting into a recess (37) in the shaft, the radial outwards movement of the locking member (24) being limited by a stop (21) co-operating with the inner sleeve and displaceable into a position permitting the radial outwards movement of the locking member, and wherein a thickened end of the push-pull rod engages in a receiving opening (33) in a spring chuck, characterised in that the stop (21) and the spring chuck (29) are displaceable into their respective release positions by a common actuating member.

2. A tubular-shaft surgical instrument according to claim 1, characterised in that the locking member (24) is a ball.

3. A tubular-shaft surgical instrument according to claim 1 or 2, characterised in that the recess (37) in the shaft (1) is bounded in the circumferential direction and in the longitudinal direction of the shaft (1), and thus the shaft (1) is fixed in the axial direction and in the circumferential direction when the locking member (24) engages in the recess (37).

4. A tubular-shaft surgical instrument according to claim 3, characterised in that a plurality of recesses (37) are arranged around the circumference of the shaft (1).

5. A tubular-shaft surgical instrument according to any one of the preceding claims, characterised in that the stop is formed by an outer sleeve (16) surrounding the inner sleeve (14) and displaceable thereon, the outer sleeve (16) having a region (21) of small inner diameter and a region (22) of larger inner diameter an axial distance apart.

6. A tubular-shaft surgical instrument according to claim 5, characterised in that the outer sleeve (16) is displaced by a spring (18) into a locking postion in which the region (21) of smaller inner diameter surrounds a radial channel (23) provided in the inner sleeve (14) and receiving the locking member (24).

7. A tubular-shaft surgical instrument according to claim 6, characterised in that the outer sleeve (16) is insertable into the grip (4) against the action of the spring (18).

8. A tubular-shaft surgical instrument according to any one of claims 5 to 7, characterised in that the outer sleeve (16) is the actuating member displacing the spring chuck (29).

9. A tubular-shaft surgical instrument according to claim 8, characterised in that the outer sleeve (16) has an extension (25) which rests against the spring chuck (29) and displaces it.

10. A tubular-shaft surgical instrument according to either one of claims 8 and 9, characterised in that, in the locking position, the outer sleeve (16) is at a distance from the spring chuck (29) and only comes to rest thereagainst after being displaced against the force of the spring (18) displacing the outer sleeve (16) into the locking position.

11. A tubular-shaft surgical instrument according any one of the preceding claims, characterised in that the spring chuck (29) comprises an inner part (30) having clamping jaws (32) bending resiliently outwards, and a sleeve-type outer part (31) surrounding the latter and axially displaceable relative thereto, the outer part (31) being displaced by a spring (34) into a locking position surrounding the free end of the clamping jaws (32).

12. A tubular-shaft surgical instrument according to claims 9 and 11, characterised in that the extension (25) of the outer sleeve (16) is formed by two diametrically opposing wall portions forming a space (26) inbetween in the form of a radial groove, and in that the ends of these wall portions come to rest against the sleeve-type outer part (31).

13. A tubular-shaft surgical instrument according to claim 12, characterised in that the flat inner surfaces (27) of the wall portions lie flat against a crosspiece (11) of the grip (4).

14. A tubular-shaft surgical instrument according to claim 13, characterised in that the crosspiece (11) has an external thread, by means of which it can be screwed into an internal thread (8) in a casing (7) of the grip (4).

15. A tubular-shaft surgical instrument according to claim 14, characterised in that the crosspiece (11) is connected to a cap (10) surrounding the inner and outer sleeve (14 and 16 respectively).

16. A tubular-shaft surgical instrument according to any one of claims 13 to 15, characterised in that the crosspiece (11) has a central bore (13) receiving and axially non-displaceably mounting the inner sleeve (14).

17. A tubular-shaft surgical instrument according to any one of the preceding claims, characterised in that the grip (4) is in the form of a tubular member arranged concentrically with the shaft (1) and the push-pull rod (3).

## Revendications

1. Instrument chirurgical à fût tubulaire comportant un fût (1), une tige de poussée et de traction (3) montée coulissante dans celui-ci, un manche de préhension (4) et une poignée (4) servant à faire coulisser la tige de poussée et de traction par rapport au fût, et des moyens de liaison qui relient les uns aux autres de manière détachable d'une part le manche de préhension et d'autre part le fût ainsi que la tige de poussée et de traction, le fût étant fixé en direction axiale dans un manchon (14) intérieur du manche de préhension au moyen d'un corps de blocage (24) à déplacement radial dans le manchon intérieur et plongeant dans un évidement (37) du fût, dont le mouvement radial de sortie est limité par une butée (21) coopérant avec le manchon intérieur, ladite butée étant déplaçable dans une position libérant le mouvement radial de sortie du corps de blocage, et la tige de poussée et de traction s'engageant avec une extrémité renflée dans une ouverture de réception (33) d'une pince de serrage, caractérisé en ce que la butée (21) et la pince de serrage (29) sont déplaçables dans leur position respective de libération grâce à un organe d'actionnement commun.

2. Instrument à fût tubulaire selon la revendication 1, caractérisé en ce que le corps de blocage (24) est une bille.

3. Instrument à fût tubulaire selon l'une ou l'autre des revendications 1 et 2, caractérisé en ce que l'évidement (37) du fût (1) est délimité en direction périphérique et en direction longitudinale du tut (1) de sorte qu'une fixation du fût en direction axiale et en direction périphérique se produit par engagement du corps de blocage (24) dans l'évidement (37).

4. Instrument à fût tubulaire selon la revendication 3, caractérisé en ce que plusieurs évidements (37) sont agencés le long de la périphérie du fût (1).

5. Instrument à fût tubulaire selon l'une quelconque des revendications précédentes, caractérisé en ce que la butée est formée par un manchon extérieur (16) entourant le manchon intérieur (14) et déplaçable sur celui-ci, ledit manchon extérieur présentant à distance axiale une région (21) de diamètre intérieur faible et une région (22) de diamètre intérieur plus grand.

6. Instrument à fût tubulaire selon la revendication 5, caractérisé en ce que le manchon extérieur (16) est déplacé grâce à un ressort (18) dans une position de blocage dans laquelle la région (21) présentant le diamètre intérieur plus faible entoure un canal radial (23) du manchon (14) intérieur, qui reçoit le corps de blocage (24).

7. Instrument à fût tubulaire selon la revendication 6, caractérisé en ce que le manchon extérieur (16) est susceptible d'être enfiché dans le manchon de préhension (4) à l'encontre de l'effet du ressort (18).

8. Instrument à fût tubulaire selon l'une quelconque des revendications 5 à 7, caractérisé en ce que le manchon extérieur (16) est l'organe d'actionnement qui déplace la pince de serrage (29).

9. Instrument à fût tubulaire selon la revendication 8, caractérisé en ce que le manchon extérieur (16) présente un prolongement (24) qui est en appui contre la pince de serrage (29) et déplace celle-ci.

10. Instrument à fût tubulaire selon l'une ou l'autre des revendications 8 et 9, caractérisé en ce que le manchon extérieur (16) garde dans la position de blocage une distance par rapport à la pince de serrage (29) et ne vient en appui contre celle-ci qu'après un déplacement à l'encontre de la force du ressort (18) déplaçant le manchon extérieur (16) dans la position de blocage.

11. Instrument à fût tubulaire selon l'une quelconque des revendications précédentes, caractérisé en ce que la pince de serrage (29) présente une partie intérieure (30) comportant des mâchoires de serrage (32) élastiquement flexibles vers l'extérieur et une partie extérieure (31) en forme de douille entourant la partie intérieure et à déplacement axial par rapport à celle-ci, ladite partie extérieure étant déplacée par un ressort (34) dans une position de blocage entourant les mâchoires de serrage (32) sur leur extrémité libre.

12. Instrument à fût tubulaire selon les revendications 9 et 11, caractérisé en ce que le prolongement (25) du manchon extérieur (16) est formé grâce à deux tronçons de parois diamétralement opposés qui forment entre eux un espace intermédiaire (26) sous la forme d'une rainure radiale et en ce que sur la face frontale, ces tronçons de paroi viennent en appui sur la partie extérieure (31) en forme de douille.

13. Instrument à fût tubulaire selon la revendication 12, caractérisé en ce que les faces intérieures (27) planes des tronçons de parois reposent à plat sur une traverse (11) du manche de préhension (4).

14. Instrument à fût tubulaire selon la revendication 13, caractérisé en ce que la traverse (11) porte un filetage avec lequel elle est susceptible d'être vissée dans un taraudage (8) dans un boîtier (7) du manche de préhension (4).

15. Instrument à fût tubulaire selon la revendication 14, caractérisé en ce que la traverse (11) est reliée à un capuchon (10) entourant les manchons intérieur 14 et extérieur 16, respectivement.

16. Instrument à fût tubulaire selon l'une quelconque des revendications 13 à 15, caractérisé en ce que la traverse (11) présente un perçage (13) central qui reçoit le manchon intérieur (14) et qui le maintient indéplaçable axialement.

17. Instrument à fût tubulaire selon l'une quelconque des revendications précédentes, caractérisé en ce que le manche de préhension (4) a la forme d'une pièce tubulaire qui est agencée de manière concentrique par rapport au fût (1) et à la tige de poussée et de traction (3).
